(19) Europäisches Patentamt

European Patent Office

Office européen des brevets

(11) **EP 2 822 568 B1**

(12) **EUROPEAN PATENT SPECIFICATION**

(45) Date of publication and mention
of the grant of the patent:
**17.06.2020 Bulletin 2020/25**

(21) Application number: **13757439.8**

(22) Date of filing: **11.03.2013**

(51) Int Cl.:
*A61K 35/20* (2006.01)     *A61P 1/14* (2006.01)
*A23J 1/20* (2006.01)     *A61K 38/17* (2006.01)
*A61P 1/00* (2006.01)     *A61P 3/00* (2006.01)
*A61P 3/02* (2006.01)     *A61P 3/04* (2006.01)
*A61P 3/08* (2006.01)     *A61P 7/00* (2006.01)
*A61P 21/06* (2006.01)     *A23C 9/142* (2006.01)
*A23C 9/146* (2006.01)

(86) International application number:
**PCT/NZ2013/000035**

(87) International publication number:
**WO 2013/133727 (12.09.2013 Gazette 2013/37)**

(54) **USES OF CASEIN COMPOSITIONS**

VERWENDUNGEN VON KASEINZUSAMMENSETZUNGEN

UTILISATIONS DE COMPOSITIONS À BASE DE CASÉINE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **09.03.2012 US 201261608858 P**

(43) Date of publication of application:
**14.01.2015 Bulletin 2015/03**

(73) Proprietor: **Fonterra Co-Operative Group Limited
Auckland 1010 (NZ)**

(72) Inventors:
• **FANNING, Aaron Calvin
Auckland Central
Auckland 1010 (NZ)**
• **PATEL, Hasmukh Ambalal
Auckland Central
Auckland 1010 (NZ)**
• **SCHALK, Johannes
Auckland Central
Auckland 1010 (NZ)**
• **HALL, Ramon Stafford
Auckland Central
Auckland 1010 (NZ)**
• **SCHWARZENBACH, Roger Richard
Auckland Central
Auckland 1010 (NZ)**
• **UKRAINTSEV, Sergey Yevgenievich
Auckland Central
Auckland 1010 (NZ)**

(74) Representative: **Walker, Ross Thomson
Forresters IP LLP
Skygarden
Erika-Mann-Strasse 11
80636 München (DE)**

(56) References cited:
**WO-A1-01/41578     WO-A1-01/41579
WO-A1-2004/026294     WO-A1-2004/057971
WO-A1-2012/008858     US-A1- 2002 044 988
US-A1- 2011 256 297**

• **Jose A. L. Calbet ET AL: "Gastric emptying,
gastric secretion and enterogastrone response
after administration of milk proteins or their
peptide hydrolysates in humans", EUROPEAN
JOURNAL OF NUTRITION, vol. 43, no. 3, 1 June
2004 (2004-06-01), pages 127-139, XP055526178,
DE ISSN: 1436-6207, DOI:
10.1007/s00394-004-0448-4**
• **M Fox ET AL: "Dietary protein precipitation
properties have effects on gastric emptying in
healthy volunteers", CLINICAL NUTRITION., vol.
23, no. 4, 1 August 2004 (2004-08-01) , pages
641-646, XP055526171, GB ISSN: 0261-5614, DOI:
10.1016/j.clnu.2003.10.013**

**(Cont. next page)**

- K. Van Norren: "Dose-dependent effects of leucine supplementation on preservation of muscle mass in cancer cachectic mice", Oncology Reports, 18 April 2011 (2011-04-18), XP055526279, ISSN: 1021-335X, DOI: 10.3892/or.2011.1269
- Layne E Norton ET AL: "Leucine regulates translation initiation of protein synthesis in skeletal muscle after exercise", The Journal of nutrition, 1 February 2006 (2006-02-01), pages 533S-537S, XP055526282, United States DOI: 10.1093/jn/136.2.533S Retrieved from the Internet: URL:https://academic.oup.com/jn/article-pd f/136/2/533S/23946806/533s.pdf
- Isabelle Rieu ET AL: "Leucine supplementation improves muscle protein synthesis in elderly men independently of hyperaminoacidaemia : Leucine and protein metabolism in the elderly", The Journal of Physiology, vol. 575, no. 1, 8 August 2006 (2006-08-08), pages 305-315, XP055526283, GB ISSN: 0022-3751, DOI: 10.1113/jphysiol.2006.110742

**Description**

**FIELD OF THE INVENTION**

The present invention relates to a casein composition for increasing the rate of gastric emptying following ingestion of the composition, increasing the digestibility of a protein composition, or increasing the rate of delivery of amino acids to the blood, or for increasing the blood serum concentration of free leucine in a subject, preferably to substantially the same level as whey protein, the casein being about 10 to about 100% calcium depleted, having a degree of hydrolysis less than about 1% and having an unmodified phosphorylation pattern.

**BACKGROUND OF THE INVENTION**

Skeletal muscle contains 50-75% of all protein in the human body. Substantial depletion of skeletal muscle, associated with ageing (for example, age-related sarcopenia), various disease states (for example, cachexia and anorexia) or following surgery or sports, can increase both morbidity and mortality. Age-related sarcopenia is a condition characterised by progressive and generalised loss of skeletal muscle mass and strength. This carries increased risk of adverse outcomes such as physical disability, poor quality of life and death. Cachexia is defined as severe muscle wasting and is a complex metabolic syndrome associated with underlying illness. It is characterised by a loss of muscle mass, with or without loss of fat (Cruz-Jentoft AJ, Baeyen JP, Bauer JM et al. Sarcopenia: European consensus on definition and diagnosis. Age and ageing 2010; 39:412-423).

The elderly or those suffering from disease require increased protein in their diet at a level greater than what is need for younger healthy individuals. Various studies report that additional protein intake can improve muscle mass, strength and function in the elderly. Additionally, other factors, including immune status, wound healing, blood pressure and bone health may be improved by increasing protein intake. (Wolf RR et al. Optimal protein intake in the elderly. Clin Nutr. 2008; 5:675-684).

The essential amino acids (EAAs) are primarily responsible for the regulation of muscle protein synthesis, and of the EAAs, leucine is recognised as having a particular role in the stimulation of muscle protein synthesis (Garlick PJ. The role of leucine in the regulation of protein metabolism. J Nutr 2005; 135:1553S-1556S).

Sources of leucine include meat, nuts, beans, brown rice, wheat and dairy. Dairy casein contains high levels of leucine, isoleucine and valine although whey protein contains higher levels of leucine and of total essential amino acids (Fox PF, McSweeney PLH (eds), Advanced Dairy Chemistry, Volume 1 - Proteins, 3rd Ed, Kluwer Academic/Plenum Publishers, NY, 2003). It has been reported that casein is less effective than whey at increasing the concentration of essential amino acids including leucine in the blood (Boirie, Y. et al. Slow and fast dietary proteins differently modulate postprandial protein accretion. Proc. Natl. Acad. Sci. 1997; 94(26):14930-14935).

The following technical papers are considered relevant to the technology field of the present invention: Jose A. L. Calbet ET AL: "Gastric emptying, gastric secretion and enterogastrone response after administration of milk proteins or their peptide hydrolysates in humans", EUROPEAN JOURNAL OF NUTRITION, vol. 43, no. 3, 1 June 2004 (2004-06-01), pages 127-139; M Fox ET AL: "Dietary protein precipitation properties have effects on gastric emptying in healthy volunteers", CLINICAL NUTRITION., vol 23, no. 4, 1 August 2004 (2004-08-01), pages 641-646; K. Van Norren: "Dose-dependent effects of leucine supplementation on preservation of muscle mass in cancer cachectic mice", Oncology Reports, 18 April 2011 (2011-04-18); Layne E Norton ET AL: "Leucine regulates translation initiation of protein synthesis in skeletal muscle after exercise", The Journal of Nutrition, 1 February 2006 (2006-02-01), pages 533S-537S; and Isabelle Rieu ET AL: "Leucine supplementation improves muscle protein synthesis in elderly men independently of hyperaminoacidaemia: Leucine and protein metabolism in the elderly", The Journal of Physiology, vol. 575, no. 1, 8 August 2006 (2006-08-08), pages 305-315.

It is therefore an object of the present invention to provide an improved or alternative method for increasing the rate of gastric emptying following ingestion of a casein composition, increasing the digestibility of a protein composition, increasing the rate of delivery of amino acids to the blood, or increasing the serum leucine concentration in a subject, or to at least provide the public with a useful choice.

**SUMMARY OF THE INVENTION**

Accordingly, in one aspect the present invention relates to the use of a casein composition for increasing the blood serum concentration of free leucine in a subject, wherein the casein composition comprises or the casein is in the form of a calcium depleted milk protein concentrate or isolate (MPC or MPI), the casein being 10 to 100% calcium depleted, having a degree of hydrolysis less than 1% and having an unmodified phosphorylation pattern; and wherein the use is not for the purpose of carrying out therapy on the human or animal body, and preferably being able to increase the blood serum concentration of free leucine in a subject to substantially the same level as whey protein within about 15 to about 60 minutes of administration.

The degree of hydrolysis may be determined using methods including but not limited to HPLC, SDS PAGE and reagent-based methods such as the o-phthaldialdehyde (OPA) method. In the OPA method, a thiol reagent, such as OPA, ethanediol or dithiothreitol, or derivatives thereof, are reacted with casein, leading to binding of the thiol reagent to specific amino acids within the hydrolysed protein. Thiol-bound amino acids fluoresce strongly at 450nm and the level of fluorescence is used as a quantitative measure of the degree of hydrolysis.

In another aspect the invention relates to the use of a casein composition for increasing the rate of gastric emptying following ingestion of the composition, increasing the digestibility of a protein composition, or increasing the rate of delivery of amino acids to the blood, wherein the casein composition comprises or the casein is in the form of a calcium depleted milk protein concentrate or isolate (MPC or MPI), the casein being 10 to 100% calcium depleted, having a degree of hydrolysis less than 1% and having an unmodified phosphorylation pattern; and wherein the use is not for the purpose of carrying out therapy on the human or animal body.

In another aspect the invention relates to a casein composition for the use in a)preventing or treating cachexia in a subject, b)preventing or treating sarcopenia in a subject, c)increasing the rate of recovery following surgery in a subject, or d)increasing the rate of recovery following injury in a subject, by increasing the blood serum concentration of free leucine in the subject; wherein the casein is 10 to 100% calcium depleted, has a degree of hydrolysis less than 1% and has an unmodified phosphorylation pattern; and wherein the casein composition comprises or the casein is in the form of a calcium depleted milk protein concentrate or isolate (MPC or MPI).

Any of the following embodiments, alone or in any combination of any two or more of the following embodiments, may relate to any of the above aspects.

In one embodiment the casein is in unhydrolysed form and has an unmodified phosphorylation pattern or is unhydrolysed kappa-casein and has an unmodified phosphorylation pattern.

In one embodiment the casein increases the blood serum concentration of free leucine in a subject, preferably to substantially the same level as whey protein, within about 15, 30, 45 or 60 minutes of administration, including within about 15 to about 60, about 30 to about 60 or about 45 to about 60 minutes. In this and related embodiments, the whey protein may be a whey protein concentrate (WPC), preferably a whey protein concentrate comprising about 80% or about 85% protein by weight.

In one embodiment, the casein is able to increase the concentration of free leucine in blood serum to at least about 205, 210, 215, 220, 225, 230, 235, 240, 245, 250, 255, 260, 265, 270, 275, 280, 285, 290, 295, 300, 305, 310, 315, 320, 325, 330, 335, 340, 345, 350, 355, 360, 365, 370, 375, 380, 385, 390, 395 or 400 μmol/L or more, and useful ranges may be selected between any of these values (for example about 205 to about 255, about 210 to about 255, about 215 to about 255, about 220 to about 255, about 225 to about 255, about 230 to about 255, about 235 to about 255, about 240 to about 255, about 205 to about 400, about 210 to about 400, about 215 to about 400, about 220 to about 400, about 225 to about 400, about 230 to about 400, about 235 to about 400, about 240 to about 400, about 245 to about 400, about 250 to about 400, about 255 to about 400, about 260 to about 400, about 265 to about 400, about 270 to about 400, about 275 to about 400, about 280 to about 400, about 285 to about 400, about 290 to about 400, about 295 to about 400, about 300 to about 400, about 305 to about 400, about 310 to about 400, about 315 to about 400, about 320 to about 400, about 325 to about 400, about 330 to about 400, about 335 to about 400, about 340 to about 400, about 345 to about 400 and about 350 to about 400 μmol/L).

In another embodiment, the casein is able to increase the concentration of free leucine in blood serum to at least about 205 μmol/L, as described above, for at least about 5, 10, 15, 20, 25, 30, 35, 40, 45 or 50 minutes or more following administration, and useful ranges may be selected between any of these values (for example about 5 to about 50, about 10 to about 50, about 15 to about 50, about 20 to about 50, about 25 to about 50, and about 30 to about 50 minutes).

In one embodiment, the casein is able to increase the concentration of free leucine in blood serum by at least about 100, 105, 110, 115, 120, 125, 130, 135, 140, 145, 150, 155, 160, 165, 170, 175, 180, 185, 190, 195, 200, 205, 210, 215, 220, 225, 230, 235, 240, 245, 250 or 255% or more, and useful ranges may be selected between any of these values (for example about 100 to about 255, about 105 to about 255, about 110 to about 255, about 115 to about 255, about 120 to about 255, about 125 to about 255, about 130 to about 255, about 135 to about 255, about 140 to about 255, about 145 to about 255, about 150 to about 255, about 155 to about 255, about 160 to about 255, about 165 to about 255, about 170 to about 255, about 175 to about 255, about 180 to about 255, about 185 to about 255, about 190 to about 255, about 195 to about 255, about 200 to about 255, about 205 to about 255, about 210 to about 255, about 215 to about 255, about 220 to about 255, about 225 to about 255, about 230 to about 255, about 235 to about 255, and about 240 to about 255%).

In another embodiment, the casein is able to increase the concentration of free leucine in blood serum by at least about 100 to at least about 205% or more, as described above, for at least about 5, 10, 15, 20, 25, 30, 35, 40, 45 or 50 minutes or more following administration, and useful ranges may be selected between any of these values (for example about 5 to about 50, about 10 to about 50, about 15 to about 50, about 20 to about 50, about 25 to about 50, and about 30 to about 50 minutes).

In one embodiment, the casein is able to increase the concentration of free leucine in blood serum by at least about 105, 110, 115, 120, 125, 130, 135, 140, 145, 150, 155, 160, 165, 170, 175, 180, 185, 190, 195, 200, 205, 210, 215, 220, 225, 230, 235, 240, 245 or 250 μmol/L or more, and useful ranges may be selected between any of these values (for example about 100 to about 250, about 105 to about 250, about 110 to about 250, about 115 to about 250, about 120 to about 250, about 125 to about 250, about 130 to about 250, about 135 to about 250, about 140 to about 250, about 145 to about 250, about 150 to about 250, about 155 to about 250, about 160 to about 250, about 165 to about 250, about 170 to about 250, about 175 to about 250, about 180 to about 250, about 185 to about 250, about 190 to about 250, about 195 to about 250, about 200 to about 250, about 205 to about 250, about 210 to about 250, about 215 to about 250, about 220 to about 250, about 225 to about 250, about 230 to about 250, about 235 to about 250, and about 240 to about 250 $\mu$mol/L).

In another embodiment, the casein is able to increase the concentration of free leucine in blood serum by at least about 105 $\mu$mol/L or more, as described above, for at least about 5, 10, 15, 20, 25, 30, 35, 40, 45 or 50 minutes or more following administration, and useful ranges may be selected between any of these values (for example about 5 to about 50, about 10 to about 50, about 15 to about 50, about 20 to about 50, about 25 to about 50, and about 30 to about 50 minutes).

In various embodiments the casein remains soluble at a pH of about 1 to about 5, remains soluble in the gastrointestinal tract, remains soluble in gastrointestinal fluid, does not form a coagulum at a pH of about 1 to about 5, or does not form a coagulum in the gastrointestinal tract.

In one embodiment the casein is substantially free of bound calcium.

In one embodiment the casein comprises casein particles of about 40, 50, 60, 70, 50, 90, 100, 110, 120, 130, 140, 150, 160 or 165nm in diameter, and useful ranges may be selected between any of these values (for example about 50 to about 165, about 50 to about 100 or about 50 to about 70nm).

In various embodiments the casein composition comprises a calcium depleted casein composition; calcium depleted skim milk; calcium depleted skim milk powder; calcium depleted whole milk; calcium depleted whole milk powder; caseinate, sodium caseinate, potassium caseinate, zinc caseinate, magnesium caseinate; a milk protein concentrate or isolate that has been modified to dissociate casein micelles; non-micellar casein; a casein ingredient, such as an MPC or MPI, where at least a portion of the calcium or phosphate or both the calcium and phosphate has been replaced with sodium, potassium, zinc, magnesium and like, or a combination of any two or more thereof; an acid soluble casein; a non-micellar caseinate; chelated casein micelles; a charge-modified casein; a glycosylated casein; a casein modified to decrease its ability to form a coagulum at acid pH, preferably at a pH below pH 5, pH 4, or pH 3; a casein modified to speed its ability to be hydrolysed in the gastrointestinal tract; a casein ingredient with an altered ratio of casein to whey; a lactic acid casein; a mineral acid casein; one or more casein fractions; an alpha-casein fraction; a beta-casein fraction; a kappa-casein fraction; or any combination of any two or more thereof.

In one embodiment the calcium depleted casein composition, such as a calcium depleted milk protein concentrate, calcium depleted milk protein isolate or calcium depleted caseinate is at least about 10, 20, 30, 35, 40, 45, 50, 55, 60, 65, 70, 75, 80, 85, 90, 95, 99 or 100% calcium depleted, and useful ranges may be selected between any of these values (for example, about 30 to about 100, about 40 to about 100, about 50 to about 100, about 60 to about 100, or about 70 to about 100%).

In one embodiment the calcium depleted casein composition, such as a calcium depleted milk protein concentrate, calcium depleted milk protein isolate or calcium depleted caseinate contains about or less than about 0, 0.01, 0.05, 0.1, 0.2, 0.3, 0.4, 0.5, 0.6, 0.7, 0.8, 0.9, 1, 1.25, 1.5, 1.75, 2, 2.25, 2.5, 2.75 or 3 grams of calcium per 100 grams of casein, and useful ranges may be selected between any of these values (for example, about 0 to about 3, about 0 to about 2.5, about 0 to about 2, about 0 to about 1.5, about 0 to about 1, about 0 to about 0.8 or about 0 to about 0.5 g calcium/100 g casein).

In one embodiment the casein is stable at a pH of about 1 to about 5.

In one embodiment, the increased blood serum leucine concentration results in any one or more of the following, or any combination of any two or more thereof, preferably to substantially the same level as whey protein:

    a) maintained or increased muscle protein synthesis,
    b) maintained or increased muscle mass,
    c) prevention or reduction of loss of muscle mass,
    d) maintained or increased growth,
    e) preventing or decreasing muscle catabolism,
    f) prevention or treatment of cachexia,
    g) prevention or treatment of sarcopenia,
    h) increased rate of glycogen resynthesis,
    i) modulation of blood sugar levels,
    j) increased insulin response to raised blood glucose concentration,
    k) increased satiety,
    l) reduced satiation,
    m) reduced food intake,
    n) reduced calorie intake,
    o) improved glucose metabolism,
    p) increased rate of recovery following surgery,
    q) increased rate of recovery following injury,
    r) increased rate of recovery following exercise, or
    s) increased sports performance.

In various embodiments the composition or formulation comprises at least about 0.01, 0.1, 0.5, 5, 10, 15, 20, 25, 30, 35, 40, 45, 50, 55, 60, 65, 70, 75, 80, 85, 90, 95 or 99% by weight protein and useful ranges may be selected between any of these values (for example, about 0.01 to about 10, about 0.01 to about 20, about 0.01 to about 30, about 0.01 to about 40, about 0.01 to about 50, about 0.01 to about 60, about 0.01 to about 70, about 0.01 to about 80, or about 0.01 to about 99%).

In one embodiment the protein is from an animal or plant source or a combination thereof. In one embodiment the protein comprises casein and an additional protein source, such as animal protein or plant protein or a combination thereof. Suitable sources of animal protein include meat and whey protein. Suitable sources of plant protein include cereal, pulse, legume (such as pea, bean, lentil and soy protein), fruit, nut, and seed protein, or any combination of any two or more thereof.

In various embodiments the composition or formulation comprises at least about 0.01, 0.1, 0.5, 5, 10, 15, 20, 25, 30, 35, 40, 45, 50, 55, 60, 65, 70, 75, 79, 81, 85, 90, 95 or 99% by weight casein and useful ranges may be selected between any of these values (for example, about 0.01 to about 10, about 0.01 to about 20, about 0.01 to about 30, about 0.01 to about 40, about 0.01 to about 50, about 0.01 to about 60, about 0.01 to about 70, about 0.01 to about 80, or about 0.01 to about 99%).

In various embodiments of a composition or formulation containing protein as described above, the protein is at least about 0.01, 0.1, 0.5, 5, 10, 15, 20, 25, 30, 35, 40, 45, 50, 55, 60, 65, 70, 75, 79, 81, 85, 90, 95 or 99% by weight casein and useful ranges may be selected between any of these values (for example, about 0.01 to about 10, about 0.01 to about 20, about 0.01 to about 30, about 0.01 to about 40, about 0.01 to about 50, about 0.01 to about 60, about 0.01 to about 70, about 0.01 to about 80, or about 0.01 to about 99% casein).

In various embodiments of a composition or formulation containing protein as described above, the protein is at least about 0.01, 0.1, 0.5, 5, 10, 15, 20, 25 or 30% by weight whey protein and useful ranges may be selected between any of these values (for example, about 0.01 to about 10, about 0.01 to about 20, about 0.01 to about 30% whey protein).

In various embodiments of a composition or formulation containing protein as described above, the protein is at least about 0.01, 0.1, 0.5, 5, 10, 15, 20, 25 or 30% by weight plant protein and useful ranges may be selected between any of these values (for example, about 0.01 to about 10, about 0.01 to about 20, about 0.01 to about 30% plant protein). Suitable sources of plant protein include cereal, pulse, legume (such as pea, bean, lentil and soy protein), fruit, nut, and seed protein, or any combination of any two or more thereof.

In one embodiment the composition may additionally comprise a source of amino acids, amino acid precursors or amino acid metabolites or any combination of any two or more thereof, preferably free amino acids, amino acid precursors or amino acid metabolites, such as those selected from the group comprising alanine, arginine, asparagine, aspartic acid, cysteine, glutamic acid, glutamine, glycine, histidine, isoleucine, leucine, lysine, methionine, phenylalanine, proline, serine, threonine, tryptophan, tyrosine, valine, essential amino acids, non-essential amino acids, branched chain amino acids, amino acid precursors such as N-acetyl-L-tyrosine, amino acid metabolites such as alpha-ketoglutaric acid and non-standard amino acids, and any combination of any two or more thereof. In one embodiment the composition or formulation comprises at least about 0.01, 0.1, 0.5, 1, 5, 10 or 15% by weight or more of free amino acids amino acid, precursors or amino acid metabolites and useful ranges may be selected between any of these values (for example, about 0.01 to about 1, about 0.01 to about 5, about 0.01 to about 10, or about 0.01 to about 15%).

In one embodiment the composition or formulation comprises at least about 0.01, 0.1, 0.5, 5, 10, 15, 20, 25, 30, 35, 40, 45 or 50% by weight lipid and useful ranges may be selected between any of these values (for example, about 0.01 to about 10, about 0.01 to about 20, about 0.01 to about 30, about 0.01 to about 40, or about 0.01 to about 50%).

In one embodiment the lipid is from an animal or plant source.

In one embodiment the composition or formulation comprises at least about 0.01, 0.1, 0.5, 5, 10, 15, 20, 25, 30, 35, 40, 45, 50, 60 or 70% by weight carbohydrate and useful ranges may be selected between any of these values (for example, about 0.01 to about 10, about 0.01 to about 20, about 0.01 to about 30, about 0.01 to about 40, about 0.01 to about 50, about 0.01 to about 60, or about 0.01 to about 70%).

In one embodiment the carbohydrate is selected from monosaccharides, disaccharides, oligosaccharides, or polysaccharides, and any combination of any two or more thereof.

In one embodiment the composition or formulation comprises at least about 0.01, 0.1, 0.5, 1, 2, 3, 4, 5, 6, 7, 8, 9 or 10% by weight minerals or vitamins and useful ranges may be selected between any of these values (for example, about 0.01 to about 2, about 0.01 to about 4, about 0.01 to about 6, about 0.01 to about 8, or about 0.01 to about 10%).

In one embodiment the composition or formulation comprises from about 0.01 % to about 95% protein, from about 0.01% to about 50% lipid, from about 0.01% to about 70% carbohydrate, and from about 0.01% to about 10% minerals and vitamins. In one embodiment the composition or formulation comprises from about 0.01 % to about 50% protein, from about 0.01% to about 30% lipid, from about 0.01% to about 50% carbohydrate, and from about 0.01% to about 5% minerals and vitamins. In one embodiment the composition or formulation comprises from about 0.5% to about 15% protein, from about 0.1% to about 10% lipid, from about 0.1% to about 30% carbohydrate, and from about 0.1% to about 1% minerals and vitamins. In various embodiments the protein may comprise about 0.01 to about 99% casein, as described above.

In one embodiment the composition or formulation has an energy content from about 1 kcal (4.184 kJ) per 100ml to about 300 kcal (1255 kJ) per 100 ml.

In various embodiments the pH of the composition of formulation is about 1 to about 14, about 1 to about 5, about 6 to about 8, about 9 to 14, about 1 to about 9 or about 5 to about 9.

In one embodiment the composition or formulation further comprises any one or more of fruit juice, juice concentrates, natural or artificial flavours, natural or artificial sweeteners, natural or artificial colours, fibres, probiotics, prebiotics, herbs, caffeine, guarana, glucosamine, free amino acids (as described above), organic acids, amino acid precursors, amino acid metabolites, taurine, alpha-ketoglutaric acid, N-acetyl-L-tyrosine, creatine, creatine esters, creatine ethyl ester, creatine salts, creatine hydrochloride, beta-alanine, beta-hydroxy beta-methylbutyric acid and beta-hydroxy beta- methylbuterate, or any combination of any two or more thereof.

In one embodiment the composition or formulation is a consumer beverage that comprises from about 0.1 % to about 10% protein, from about 0.01% to about 5% lipid, from about 0.1% to about 15% carbohydrate, and from about 0.1% to about 2% minerals and vitamins and has an energy content of at least 1 kcal (4.184 kJ) per 100 ml. In one embodiment the protein may comprise about 0.01 to about 99% casein, as described above.

In one embodiment the composition or formulation is a recovery beverage additionally comprising any one or more of natural or artificial flavours, natural or artificial sweeteners, natural or artificial colours, or glucosamine, or any combination of any two or more thereof. In one embodiment the recovery beverage comprises from about 0.1% to about 10% protein, from about 0.01% to about 4% lipid, from about 0.1% to about 20% carbohydrate, and from about 0.1% to about 2% minerals and vitamins and has an energy content of at least 1 kcal (4.184 kJ) per 100 ml. In one embodiment the protein may comprise about 0.01 to about 99% casein, as described above.

In one embodiment the composition or formulation is a medical food additionally comprising any one or more of natural or artificial flavours, natural or artificial sweeteners or natural or artificial colours, or any combination of any two or more thereof. In one embodiment the medical food comprises from about 1% to about 20% protein, from about 1% to about 20% lipid, from about 1% to about 40% carbohydrate, and from about 0.1% to about 5% minerals or vitamins and has an energy content of at least 50 kcal (209 kJ) per 100 ml. In various embodiments the medical food is acidic or neutral. In one embodiment the protein may comprise about 0.01 to about 99% casein, as described above.

In various embodiments the composition or formulation is an ingredient used in the preparation of a food. In one embodiment the composition or formulation is formulated for separate, simultaneous or sequential administration with a food. In various embodiments the food is confectionary, a bar, a dairy product, milk, a milk product, a milk powder, reconstituted milk, cultured milk, yoghurt, drinking yoghurt, set yoghurt, a beverage, a food additive, a drink additive, a dietary supplement, a nutritional product, a dietetic product or a medical food.

In another embodiment the composition is or is formulated as a food, drink, food additive, drink additive, dietary supplement, nutritional product, dietetic product, medical food, enteral feeding product, parenteral feeding product, meal replacement, nutraceutical, medicament, or pharmaceutical.

In one embodiment the whey protein is a whey protein concentrate or a whey protein isolate. In another embodiment the whey protein is a whey protein concentrate or a whey protein isolate comprising about 50, 60, 70, 80 or 90% protein by weight, and useful ranges may be selected between these values (for example, about 50 to about 90%).

It is intended that reference to a range of numbers disclosed herein (for example, 1 to 10) also incorporates reference to all rational numbers within that range (for example, 1, 1.1, 2, 3, 3.9, 4, 5, 6, 6.5, 7, 8, 9 and 10) and also any range of rational numbers within that range (for example, 2 to 8, 1.5 to 5.5 and 3.1 to 4.7) and, therefore, all sub-ranges of all ranges expressly disclosed herein are hereby expressly disclosed. These are only examples of what is specifically intended and all possible combinations of numerical values between the lowest value and the highest value enumerated are to be considered to be expressly stated in this application in a similar manner.

In this specification where reference has been made to patent specifications, other external documents, or other sources of information, this is generally for the purpose of providing a context for discussing the features of the invention. Unless specifically stated otherwise, reference to such external documents is not to be construed as an admission that such documents, or such sources of information, in any jurisdiction, are prior art, or form part of the common general knowledge in the art.

**BRIEF DESCRIPTION OF THE DRAWINGS**

Figure 1 is a graph showing the change in absolute blood serum concentration of leucine ($\mu$mol/L) up to 180 minutes after consumption of (A) a calcium-depleted milk protein concentrate, (B) a whey protein concentrate from cheddar cheese manufacture, (C) a standard milk protein concentrate, and (D) a calcium caseinate.

Figure 2 is a graph showing the area under the curve of Figure 1 for the period of 0 to 180 minutes.

Figure 3 is a graph showing the area under the curve of Figure 1 for the period of 0 to 60 minutes.

Figure 4 is a graph showing the peak leucine concentration from Figure 1.

Figure 5 is a graph showing the change in absolute blood serum concentration of leucine ($\mu$mol/L) up to 90 minutes after consumption of (A) a whey protein concentrate from cheddar cheese manufacture, (B) a calcium-depleted milk protein concentrate, (C) a standard milk protein concentrate, and (D) a sodium caseinate.

Figure 6 is a graph showing the area under the curve of Figure 5 for the period of 0 to 90 minutes.

Figure 7 is a graph showing the peak leucine concentration from Figure 5.

Figure 8 is a graph showing the viscosity (Pa/s) of sodium caseinate (A), calcium-depleted MPC (B) or standard MPC (C) over time in a simulated gastric model.

## DETAILED DESCRIPTION OF THE INVENTION

The present inventors have surprisingly determined that casein ingredients may be modified to behave like whey, and so used to, amongst other things, effectively deliver leucine to the bloodstream at levels equivalent to whey. Thus the present invention relates to use of casein compositions for increasing the rate of gastric emptying following ingestion of a casein composition, increasing the digestibility of a protein composition, increasing the rate of delivery of amino acids to the blood, or increasing blood serum leucine concentration, and in particular the present invention relates to use of a casein composition for increasing the blood serum concentration of free leucine in a subject to substantially the same level as whey protein.

## 1. Definitions

The phrase "calcium depleted" is used herein to refer to a casein composition, such as a milk protein concentrate (MPC), in which the concentration of calcium bound to casein has been reduced and is lower than the concentration of calcium bound to casein in the corresponding non-depleted composition. Such a composition may also be depleted in divalent cations, and so have a lower concentration of divalent cations bound to casein, for example, magnesium, than the corresponding non-depleted composition. Similarly, reference to calcium in casein protein is a reference to bound calcium - that is, calcium bound by the casein protein.

The term "comprising" as used in this specification means "consisting at least in part of". When interpreting statements in this specification which include that term, the features, prefaced by that term in each statement or claim, all need to be present but other features can also be present. Related terms such as "comprise" and "comprised" are to be interpreted in the same manner.

The term "dietetic product" means a product specially processed or formulated to satisfy particular dietary requirements which exist because of a particular physical or physiological condition and/or specific diseases and disorders and which are presented as such.

An "effective amount" is the amount required to confer therapeutic effect. The interrelationship of dosages for animals and humans (based on milligrams per meter squared of body surface) is described by Freireich, et al., 1966. (see Freireich EJ, Gehan EA, Rall DP, Schmidt LH, Skipper HE (1966) Quantitative comparison of toxicity to anticancer agents in mouse, rat, hamster, dog, monkey and man. Cancer Chemother Rep 50: 219-244). Body surface area can be approximately determined from height and weight of the subject. See, e.g., Scientific Tables, Geigy Pharmaceuticals, Ardley, New York, 1970, 537. Effective doses also vary, as recognized by those skilled in the art, dependent on route of administration, carrier usage, and the like.

The phrase "increasing the blood serum concentration of leucine" and its grammatical equivalents and derivates, refers to maintaining and increasing blood serum leucine concentrations, preferably to adequate physiological concentrations to maximally stimulate muscle protein synthesis in the post-prandial period. For the purposes of determining leucine levels in the blood, references to "blood", "blood serum" and "blood plasma" are interchangeable.

The term "milk protein concentrate" (or MPC) is a milk protein product in which greater than 55%, preferably greater than 75% of the dry matter is milk protein and the ratio of casein to whey proteins is approximately that of milk. Such concentrates are known in the art.

The phrase "maintaining or increasing muscle mass" and its grammatical equivalents and derivatives, refers to an increase in muscle protein synthesis and/or a decrease in muscle protein breakdown which results in a gain or maintained muscle mass.

The phrase "preventing or reducing loss of muscle mass" and its grammatical equivalents and derivatives, refers to prevention or decrease in muscle protein breakdown resulting in maintained muscle mass or decreased rate of muscle loss.

A "subject" is an animal, preferably a mammal, more preferably a mammalian companion animal or human. Preferred companion animals include cats, dogs and horses. In one embodiment the subject is a human. Preferably the human is an adult, a child, or an infant.

The phrase "substantially free of bound calcium" means that a casein composition contains less than about 0.3, 0.275, 0.25, 0.225, 0.2, 0.175, 0.15, 0.125, 0.1, 0.075, 0.05, 0.025 or 0.01 % by weight calcium bound to a casein polypeptide, and useful ranges may be selected between any of these values (for example, about 0.01 to about 0.2, about 0.01 to about 0.175, about 0.01 to about 0.15, about 0.01 to about 0.125, about 0.01 to about 0.1, or about 0.01 to about 0.075%). The degree of calcium binding in casein micelles, and therefore the degree of calcium depletion of calcium depleted casein compositions may be determined by known methods, including ultrafiltration or ultracentrifugation to obtain a ca-

sein fraction followed by atomic absorption spectrometry (see for example, Philippe, M. et al, The effects of different cations on the physicochemical characteristics of casein micelles, Food Chemistry 90 (2005) 673-683, incorporated herein by reference).

The term "treat" and its derivatives should be interpreted in their broadest possible context. The term should not be taken to imply that a subject is treated until total recovery. Accordingly, "treat" broadly includes amelioration and/or prevention of the onset of the symptoms or severity of a particular condition.

The phrase "unmodified phosphorylation pattern" is intended to mean that the casein is not subjected to dephosphorylation. In one embodiment the unmodified phosphorylation pattern is the naturally occurring phosphorylation pattern. Phosphorylation patterns of casein polypeptides are reported in the art (see, for example, Fox PF, McSweeney PLH (eds), Advanced Dairy Chemistry, Volume 1 - Proteins, 3rd Ed, Kluwer Academic/Plenum Publishers, NY, 2003, incorporated herein by reference). Naturally occurring bovine alpha s1 casein has 8 or 9 phosphate residues per mole (alpha s1-CN 8P or 9P), bovine alpha s2 casein has 10, 11, 12 or 13 phosphate residues per mole (alpha s2-CN 10P, 11P, 12P, or 13P), bovine beta casein has 4 or 5 phosphate residues per mole (beta-CN 4P or 5P), and bovine kappa casein has 1, 2 or 3 phosphate residues per mole (kappa-CN 1P, 2P or 3P).

## 2. Casein

Bovine casein is the product of four genes - alpha-sl (with variants A, B, C, D, E), alpha-s2 (with variants A, C, D), beta (with variants A1, A2, A3, B, C, D, E, F) and kappa (with variants A, B, C, E, F, F1). Each casein polypeptide has a unique composition and structure. In various embodiments, casein useful herein may be bovine, camelid, caprine, cervine, equine, ovine or porcine casein, or any combination of any two or more thereof. Casein proteins and production of useful casein compositions is discussed extensively by Fox & McSweeney, 2003 (Fox PF, McSweeney PLH (eds), Advanced Dairy Chemistry, Volume 1 - Proteins, 3rd Ed, Kluwer Academic/Plenum Publishers, NY, 2003) and in the Dairy Processing Handbook, 2003 (Dairy Processing Handbook, Second Edition, Tetra Pak Processing Systems AB, Sweden, 2003.

Casein for use in any of the compositions described herein includes casein in the form of non-micellar casein, non-micellar caseinate (including sodium and potassium caseinate), lactic acid casein, mineral acid casein, $\alpha$-casein, $\beta$-casein, kappa-casein, a casein fraction, an alpha-casein fraction, a beta-casein fraction, a kappa-casein fraction, casein treated by ultra high-pressure (UHP) processing, translucent casein or any combination of any two or more thereof.

Casein useful herein has an unmodified phosphorylation pattern, as described above. In relation to bovine alpha s1-caseins B, C, D and E, for example, post-translational phosphorylation occurs at serine residues 41, 46, 48, 64, 66, 67, 68, and 115, and at threonine residue 53 in alpha s1-casein D. The phosphorylation patterns of all of the bovine caseins are reported by Fox & McSweeney, 2003.

Calcium-depleted casein compositions may be prepared by the methods described in published international PCT application WO 2001/041578 and published international PCT application WO 2004/057971. For example, one method of preparing a calcium-depleted casein composition comprises (a) providing an MPC or MPI having at least 70% dry matter as milk protein in aqueous solution/suspension; (b) removing 20-100% of calcium ions therein by a method chosen from at least one of (1) cation exchange on an ion exchanger in the sodium and/or potassium form, (2) acidification to pH <7 with subsequent dialysis and/or ultrafiltration and/or diafiltration or (3) by addition of a chelating agent; and/or binding a proportion of calcium ions with a chelating agent; (c) optionally mixing the product from step (b) with another milk solution while maintaining at least 30% calcium depletion; (d) optionally heating the solution at a temperature and for a time sufficient to allow denaturation of whey proteins and interaction with casein (for example, heating for 4-15 min at >100 °C), and (e) optionally drying to prepare a dried product. In one embodiment the calcium-depleted casein composition is at least about 10, 20, 30, 35, 40, 45, 50, 55, 60, 65, 70, 75, 80, 85, 90, 95, 99 or 100% calcium depleted, and useful ranges may be selected between any of these values (for example, about 30 to about 100%). The degree of calcium depletion of calcium depleted casein compositions may be determined by known methods, including ultrafiltration or ultracentifugation to obtain a casein fraction followed by atomic absorption spectrometry (see for example, Philippe, M. et al, The effects of different cations on the physicochemical characteristics of casein micelles, Food Chemistry 90 (2005) 673-683).

In those embodiments in which calcium removal is by acidification and subsequent dialysis and/or ultrafiltration and/or diafiltration, the pH is adjusted to be in the range pH 4.6- 6, or pH 4.8-5.5. The membrane chosen generally has a nominal molecular weight cut off of 10,000 Daltons or less. An example of a useful ultrafiltration membrane is a Koch S4 HFK 131 type membrane with a nominal molecular weight cut off at 10,000 Daltons. The adjustment of the pH may be made with any acid suitable for adjusting the pH of a food or drink e.g., dilute HCl, dilute $H_2SO_4$, dilute acetic acid, and dilute citric acid.

When the calcium removal is by way of addition of a chelating agent, preferred chelating agents for use include citric acid, EDTA, food phosphates/polyphosphates, food acidulants, tartaric acid, citrates and tartrates. In one embodiment the chelating agents are food acidulating agents. In one embodiment the chelating agents are used in conjunction with dialysis and/or ultrafiltration and diafiltration.

In one embodiment the cation exchangers are based on resins bearing strongly acidic groups, such as sulphonate groups. In one embodiment the strong acid cation exchange resin is IMAC HP 111 E manufactured by Rohm & Haas. This resin has a styrene divinylbenzene copolymer matrix. The functional groups are sulphonic acid groups that can be obtained in the Na$^+$ form or alternatively converted to the K$^+$ form.

In one embodiment, the calcium-depleted casein composition is cold soluble. The term "cold solubility" or "cold soluble" refers to the property a product which on reconstitution into a 5% w/v solution at 20 °C provides less than 5% sediment on centrifugation for 10 minutes at 700g.

Acid casein is formed by acidifying skim milk to the isoelectric point of casein - pH 4.6, resulting in precipitation of casein from the milk. Mineral acid casein is formed by acidification of skim milk by mineral acid to a pH of between 4.3 and 4.6. Lactic acid casein is produced by acidification of milk using lactic acid bacteria. The milk is cooled to a temperature of approx 23-27°C and the pH of the milk is lowered gradually. At the desired pH the milk is heated to 50-55°C, and the casein collected. See Fox & McSweeney, 2003 and the Dairy Processing Handbook, 2003.

Caseinate is a composition comprising both casein and a monovalent or divalent metal ion. Hydroxide solutions comprising sodium, potassium and magnesium ions are used to produce sodium caseinate, potassium or magnesium caseinate. See Fox & McSweeney, 2003 and the Dairy Processing Handbook, 2003.

UHP-processed translucent casein is produced by subjecting a casein-containing composition to a high pressure treatment, as described in international patent application WO 2004/091309, incorporated herein by reference in its entirety. In one embodiment the casein composition has been subjected to a method of enhancing its clarity and / or stability properties by subjecting the composition to a pressure treatment of at least about 100 MPa to obtain a composition that has a desired clarity, as determined by measuring its optical density (OD) at 610nm. A solution having a reading of zero OD passes 100% of the incident light, while a sample having an OD of 1.0 passes 10% of the incident light, and so forth. A solution with an OD of 1.0 is generally considered to be "opaque".

Translucent casein may also be produced via cation exchange of skim milk, milk protein concentrate or milk protein isolate using the methods described and exemplified in international patent application WO2001/041579. For example, one suitable method comprises a method of preparing a translucent milk drink having a pH in the range 5.6-7.0, the method comprising (a) providing an opaque milk starting material having a pH in the range 5.7-6.5; (b) contacting at least a portion of the starting material with a cation exchanger until the percentage transmission of the material (when separated from the exchanger) rises to at least 5%, preferably at least 25%, more preferably at least 40%; (c) optionally mixing the translucent milk sample with another milk sample while retaining the percentage transmission at least 5%, preferably at least 25%, more preferably at least 40%. Translucency of milk may be measured with a Turbiscan MA 2000 Macroscopic Analyser (Formulaction, Toulouse, France) using transmission of a pulsed near infrared (NIR) light source ($\lambda$ = 850 nm). The sample is contained in a special sample cell and NIR is passed through the sample and a transmission detector receives the light, which goes through the sample. The transmission detector acquires the transmitted light flux (in %) as a function of die sample height (65 mm). For the definition of translucency, an average value of transmission from 20 mm to 50 mm height of the sample cell is taken. The path length is 1cm.

## 3. Compositions

A composition useful herein may be formulated as a food, drink, food additive, drink additive, dietary supplement, nutritional product, dietetic product, medical food, enteral feeding product, parenteral feeding product, meal replacement, nutraceutical, medicament, or pharmaceutical. Appropriate formulations may be prepared by an art skilled worker with regard to that skill and the teaching of this specification.

In one embodiment the composition is acidic. In another embodiment the composition is neutral. In various embodiments the composition is formulated for oral administration.

In various embodiments the composition is any edible consumer product which is able to carry protein. Examples of suitable edible consumer products include liquids, confectionary products including, gels, ice creams, reconstituted fruit products, snack bars, food bars, muesli bars, spreads, sauces, dips, dairy products including yoghurts and cheeses, mayonnaise/salad dressings, drinks including dairy and non-dairy based drinks (such as milk drinks and yogurt drinks), sports supplements including dairy and non-dairy based sports supplements, food additives, and dietary supplement products.

In one embodiment the food is selected from confectionary, a bar, a dairy product, milk, a milk product, a milk powder, reconstituted milk, cultured milk, yoghurt, drinking yoghurt, set yoghurt, a beverage, a food additive, a drink additive, a dietary supplement, a nutritional product, a dietetic product or a medical food.

In one embodiment the composition is in the form of a cachet, a powder, a dispensable powder, granules, a suspension, an elixir, a liquid, or any other form that can be added to food or drink, including for example water, milk or fruit juice.

In one embodiment the composition is formulated as a beverage. In one embodiment the beverage is acidic. In another embodiment the beverage is neutral. In one embodiment the pH of the composition is from about 1 to about 4.6. Preferably the pH of the composition if from about 2 to about 3.7. In another embodiment the pH of the composition is about 1 to about 14, about 1 to about 5, about 6 to about 8, about 9 to 14, about 1 to about 9 or about 5 to about 9.

In embodiments where the composition is a liquid at acid pH, casein may be dissolved and hydrated in water and acid quickly added to lower the pH to 3.0 with vigorous stirring. The pH will pass through the isoelectric point of casein and the casein will re-solubilise.

In one embodiment the composition is in the form of a medical food, further comprising a source of lipid, a source of carbohydrate, optionally an additional source of protein, and optionally a source of vitamins and minerals.

The lipid used may be plant lipid or animal lipid, including dairy lipid and fish oils. Plant oils are often exemplary because of their ease of formulation and lower saturated fatty acid content. Exemplary plant oils include canola (rapeseed) oil, corn oil, sunflower oil, olive or soybean oil.

The carbohydrate used typically comprises digestible carbohydrate as 75-100% of the carbohydrate. The carbohydrate may comprise monosaccharides, disaccharides, oligosaccharides and polysaccharides and mixtures thereof. Oligosaccharides of glucose are typically used. A number of these are commercially available as maltodextrin (3-20 DE) or corn syrup for the longer chain carbohydrates (>20 DE). Non-digestible carbohydrates may also be included, for example, fructooligosaccharides, inulin, and galactooligosaccharides. These are typically present in amounts of 0.2-5% of the composition.

In exemplary embodiments die liquid nutritional composition is a nutritionally complete composition or a high energy liquid or powder for breakfast or other times of the day.

In exemplary embodiments the liquid nutritional composition contains nutrients that include vitamins and minerals. The recommended daily requirements of vitamins and minerals can be specified for various population subgroups. See for instance, Dietary Reference Intakes: RDA and AI for vitamins and elements, United States National Academy of Sciences, Institute of Medicine, Food and Nutrition Board (2010) tables recommended intakes for infants 0-6, 6-12 months, children 1-3, and 4-8 years, adults males (6 age classes), females (6 age classes), pregnant (3 age classes) and lactating (3 age classes). Concentrations of essential nutrients in the liquid nutritional composition can be tailored in the exemplary serve size for a particular subgroup or medical condition or application so that the nutrition and ease of delivery requirements can be met simultaneously.

In one embodiment the composition is in the form of pharmaceutical composition comprising an appropriate pharmaceutically acceptable carrier (including excipients, diluents, auxiliaries, and combinations thereof) selected with regard to the intended route of administration and standard pharmaceutical practice. See for example, Remington's Pharmaceutical Sciences, 16th edition, Osol, A. Ed., Mack Publishing Co., 1980.

Accordingly, the invention also is directed to doses, dosage forms, formulations, compositions comprising casein, including those disclosed herein, useful for the therapy of diseases, disorders, and/or conditions in humans and other mammals and other disorders as disclosed herein. The use of these dosage forms or formulations comprising one or more compositions of the invention enables effective treatment of these conditions. The dosage forms, formulations or compositions of the invention may be formulated to optimize bioavailability, or to return or maintain plasma, blood, or tissue concentrations within the normal therapeutic range, including for extended periods.

The dosage forms, formulations or compositions of the invention may be formulated for periodic administration, for example to provide continued exposure to the one or more compositions of the invention.

Examples of dosage forms suitable for oral administration include, but are not limited to tablets, capsules, lozenges, or like forms, or any liquid forms such as syrups, aqueous solutions, emulsions and the like, capable of providing a therapeutically effective amount of casein.

Further examples of dosage forms of the invention include, but are not limited to modified-release (MR) dosage forms including delayed-release (DR) forms; prolonged-action (PA) forms; controlled-release (CR) forms; extended-release (ER) forms; timed-release (TR) forms; and long-acting (LA) forms. For the most part, these terms are used to describe orally administered dosage forms, however these terms may be applicable to any of the dosage forms, formulations or compositions described herein. These formulations effect delayed total drug release for some time after drug administration, and/or drug release in small aliquots intermittently after administration, and/or drug release slowly at a controlled rate governed by the delivery system, and/or drug release at a constant rate that does not vary, and/or drug release for a significantly longer period than usual formulations.

The compositions useful herein may be used alone or in combination with one or more additional agents. The additional agent may be a food, drink, food additive, drink additive, dietary supplement, nutritional product, dietetic product, medical food, enteral feeding product, parenteral feeding product, meal replacement, nutraceutical, medicament, or pharmaceutical. The additional agent may increase the efficacy at which casein is broken down or leucine is delivered to the bloodstream.

When used in combination with an additional agent, the administration of a composition useful herein and the other agent may be simultaneous or sequential. Simultaneous administration includes the administration of a single dosage form that comprises all components or the administration of separate dosage forms at substantially the same time. Sequential administration includes administration according to different schedules, preferably so that there is an overlap in the periods during which the composition useful herein and other therapeutic agent are provided.

Additionally, it is contemplated that a composition in accordance with the invention may be formulated with additional active ingredients which may be of benefit to a subject in particular instances. For example, agents, such as therapeutic agents that target the same or different facets of the condition or disease process or interest may be used. Suitable agents are described above.

In one embodiment the additional active ingredients may include lipids, carbohydrates, other proteins, minerals or vitamins, or flavouring agents.

In one embodiment the composition may additionally comprise a source of amino acids, such as free amino acids, as described above.

In one embodiment the composition may be supplemented with minerals, including, but not limited to chloride, sodium, calcium, iron, chromium, copper, iodine, zinc, magnesium, manganese, molybdenum, phosphorus, potassium, and selenium. Suitable forms of any of the foregoing minerals include soluble mineral salts, slightly soluble mineral salts, insoluble mineral salts, chelated minerals, mineral complexes, non-reactive minerals such as carbonyl minerals, and reduced minerals, and combinations thereof.

The compositions may also optionally comprise vitamins. The vitamins may be fat-soluble or water soluble vitamins. Suitable vitamins include but are not limited to vitamin C, vitamin A, vitamin E, vitamin B12, vitamin K, riboflavin, niacin, vitamin D, vitamin B6, folic acid, pyridoxine, thiamine, pantothenic acid, and biotin. The form of the vitamin may include salts of the vitamin, derivatives of the vitamin, compounds having the same or similar activity of a vitamin, and metabolites of a vitamin.

It should be understood that the additional agents may also be employed in a method according to the invention where they are administered separately, simultaneously or sequentially with a composition useful herein. The proportions of each component in the composition are tailored to optimise the efficacy of the composition for delivering leucine to or increasing muscle mass in a subject in need thereof.

As will be appreciated, the dose of the composition administered, the period of administration, and the general administration regime may differ between subjects depending on such variables as the severity of symptoms of a subject, the type of disorder to be treated, the mode of administration chosen, and the age, sex and/or general health of a subject. However, by way of general example, the inventors contemplate administration of from about 1 mg to about 2500 mg per kg body weight of a casein composition described herein.

It should be appreciated that administration may include a single daily dose or administration of a number of discrete divided doses as may be appropriate.

It should be understood that a person of ordinary skill in the art will be able without undue experimentation, having regard to that skill and this disclosure, to determine an effective dosage regime (including daily dose and timing of administration) for a given condition.

Compositions useful according to the invention may be prepared using methods known in the art.

Methods of preparing an acid casein beverage include dissolving casein-containing powder with water to produce a solution to which sugar, flavour and colouring are added. The mixture is cooled to less than 30 degrees Celsius and the pH adjusted to 4.1 using a blended solution of citric and lactic acids. Water is added to the desired volume and the composition is homogenised and pasteurized.

Neutral compositions are prepared by dissolving casein-containing powder with water to produce a solution, to which sugar, flavour and colouring are added. The mixture is cooled to less than 30 degrees Celsius and the solution adjusted to the desired pH. The composition is homogenised and heat treated at 140 degrees Celsius.

## 4. Therapeutic outcomes and methods of assessing therapeutic outcomes

Consumption of a composition of the invention by a subject may result in any one or more of the following, or any combination of any two or more thereof, preferably to substantially the same level as whey protein:

a) maintained or increased muscle protein synthesis,
b) maintained or increased muscle mass,
c) prevention or reduction of loss of muscle mass,
d) maintained or increased growth,
e) preventing or decreasing muscle catabolism,
f) prevention or treatment of cachexia,
g) prevention or treatment of sarcopenia,
h) increased rate of glycogen resynthesis,
i) modulation of blood sugar levels,
j) increased insulin response to raised blood glucose concentration,
k) increased satiety,
l) reduced satiation,
m) reduced food intake,
n) reduced calorie intake,
o) improved glucose metabolism,
p) increased rate of recovery following surgery,
q) increased rate of recovery following injury,
r) increased rate of recovery following exercise, or
s) increased sports performance.

The method of achieving the above effects comprises the step of administering to a subject in need thereof an effective amount of a casein composition, as described herein, according to methods as described herein. It should be understood that in some embodiments consumption of a composition of the invention by a subject may result in at least maintenance of one of the above effects. For example, maintaining muscle mass, or maintaining the blood serum concentration of leucine.

The efficacy of a composition useful according to the invention can be evaluated both *in vitro* and *in vivo*, and methods for such are known in the art. Briefly, in one embodiment a candidate composition can be tested for its ability, to for example, normalise or restore leucine levels or muscle mass. For *in vivo* studies, a composition can be fed to or administered to an animal (e.g., a mouse) and its effects on restoring leucine delivery then assessed. Based on the results, an appropriate dosage range and administration route can be determined.

Furthermore, consumption of a composition of the invention by a subject may result in any one or more of increasing the rate of gastric emptying following ingestion of the composition, increasing the digestibility of a protein composition, or increasing the rate of delivery of amino acids to the blood. Methods of assessing gastric emptying, digestibility and delivery of amino acids are known in the art.

### 4.1 Increased delivery of leucine

Assessment of blood serum leucine concentration following a meal is an important factor for assessing the ability of a protein-rich meal to stimulate muscle protein metabolism. Assessment of blood serum leucine following a meal is achieved by taking and assaying baseline blood samples, and serially collecting samples in the post-prandial period (up to 3 hours post prandial).

Blood serum leucine concentration may be assessed according to the methods of the examples below, or as follows. Blood samples are collected in heparinised vacutainers. A $100\mu$L of whole blood is added to cold perchloric acid, and chilled for 10 minutes to allow the proteins to precipitate. Following centrifugation at 4000g for 2 minutes, 250 $\mu$L of $KHCO_3$ is added to neutralise the solution. The supernatant is stored -80°C until further analysis. Analysis of amino acids is achieved by high performance liquid chromatography (HPLC) complete with suitable standards.

### 4.2 Increased and decreased muscle protein synthesis

Muscle protein synthesis is assessed by the changes in protein balance within a muscle. Various methods are available to assess changes in protein balance. Typically, methods involve measuring the fractional rate of muscle protein synthesis within a set time period (i.e. 2-3 hours post prandially). Briefly, participants have a baseline muscle biopsy taken from the vastus lateralis using standard protocols, and the muscle sample is blotted dry, visible fat and connective tissue is removed and the sample frozen in liquid nitrogen and stored at -80°C until further analysis.

After refraining from any strenuous physical activity for three days, subjects have a catheter inserted into their antecubital vein for blood sampling. Following baseline blood samples, a second catheter is inserted into their collateral arm for administration of a primed, constant, infusion of L-[ring-$^{13}C_6$]phenylalanine. Three hours following the start of the infusion, the subject undergoes a muscle biopsy from the collateral limb (from baseline measure) to measure the fasting rates of protein synthesis. Blood samples are drawn, and the subjects consume a protein beverage (enriched with 6% of the L-[ring-$^{13}C_6$]phenylalanine, to minimize disturbances in the isotopic equilibrium) to stimulate muscle protein synthesis in the post prandial period. Three hours after administration of the protein product, a final muscle biopsy is taken to assess the protein synthesis response that has occurred in the post prandial period.

Analysis of samples occurs by isolating intracellular proteins from a piece of wet muscle. The sample is spun for 10 minutes to separate myofibrillar and collagen proteins. Removal of the sarcoplasmic proteins is achieved by precipitation with perchloric acid. The myofibrillar proteins are solubilised by adding NaOH and mixing every 10 minutes. Centrifugation at 4000g creates a supernatant containing the myofibrillar proteins, which is precipitated out using perchloric acid, washed and lyophilised. The amino acids are liberated using HCl and the sample is heated at 110 °C for 24 hours. Free amino acids are purified using cation exchange chromatography, and converted to their N-Acetyl-n-propyl ester derivatives for analysis by gas chromatography combustion-isolate ratio mass spectrometry.

The fractional synthetic rate is calculated using the formulae:

$$\mathrm{FSR}\ (\%\ \mathrm{h}{-}1) = \mathrm{Ep/Em} \times 1/\mathrm{t} \times 100$$

Where 'Ep' is the change in bound protein enrichment between the two biopsy samples, 'Em' is the average enrichment of the intracellular phenylalanine across the two samples and 't' is the time between the biopsies.

### 4.3 Reduction of loss of muscle mass

Changes in muscle mass are assessed by utilising repeated measures of body composition with a suitable multi-compartment body composition analysis technique, such as Dual Energy X-ray Absorpitrometry, or a multi compartment model derived from hydrodensitometry or total body density.

Methods for assessing satiety and satiation are known in the art. Suitable methods are reported in, for example, published international patent application WO2010/107325 (PCT/NZ2010/000042) tided "Satiety Product".

**4.5 Gastric emptying and digestibility**

Methods for assessing the rate of gastric emptying and digestibility are known in the art. See, for example, L. A. Szarka and M. Camilleri, "Methods for measurement of gastric motility", Am J Physiol Gastrointest Liver Physiol 296: G461-G475, 2009, and A. J. Darragh and S. M. Hodgkinson, "Quantifying die Digestibility of Dietary Protein", J Nutr. 2000 Jul;130(7):1850S-6S,

Various aspects of the invention will now be illustrated in non-limiting ways by reference to the following examples.

**EXAMPLES**

A description of each of the ingredients used in the examples is provided below.

Standard milk protein concentrate (about 85% protein by dry weight, about 3.279 g calcium/100 g casein protein; Fonterra Co-operative Group Limited, New Zealand). This is produced by ultrafiltration and diafiltration, from skim milk (Fox PF, McSweeney PLH (eds), Advanced Dairy Chemistry, Volume 1 - Proteins, 3rd Ed, Kluwer Academic/Plenum Publishers, NY, 2003).

Calcium depleted milk protein concentrate (about 85% protein by dry weight, about 80% calcium depleted - about 0.411 g calcium/100 g casein protein; Fonterra Co-operative Group Limited, New Zealand) was produced by the methods described in published international PCT application WO 2001 041578.

Whey protein concentrate (about 80% protein by dry weight, about 0.5 g calcium/100 g whey protein; Fonterra Co-operative Group Limited, New Zealand). This is produced by ultrafiltration and diafiltration of cheese whey (Fox PF, McSweeney PLH (eds), Advanced Dairy Chemistry, Volume 1 - Proteins, 3rd Ed, Kluwer Academic/Plenum Publishers, NY, 2003).

Calcium caseinate (CaCas, about 90% protein by dry weight, about 1.5 g calcium/100 g casein protein; Fonterra Co-operative Group Limited, New Zealand) was manufactured by solubilising casein in water and adjusting the pH to 6.7 using calcium hydroxide (Fox PF, McSweeney PLH (eds), Advanced Dairy Chemistry, Volume 1 - Proteins, 3rd Ed, Kluwer Academic/Plenum Publishers, NY, 2003).

Sodium caseinate (NaCas, about 90% protein by dry weight, about 0.033 g calcium/100 g casein protein; Fonterra Co-operative Group Limited, New Zealand) was manufactured by solubilising casein in water and adjusting pH to 6.7 using sodium hydroxide(Fox and McSweeney, 2003).

**Example 1: Comparison of various protein ingredients for delivering leucine to the bloodstream.**

Each of the protein ingredients were prepared as described above.

Fifteen subjects aged 18-49 were selected to participate in the trial. Exclusion criteria for the trial included a regular use of protein supplements, a BMI of greater than $30 kg/m^2$, a history of diabetes mellitus - Type I or II, or heart disease, chronic disease of the gastrointestinal tract (for example, inflammatory bowel disease), previous gastrointestinal surgery, pregnancy, allergies to any dietary protein (such as dairy or soy), serious comorbidity that could, in the opinion of the investigator, prevent the subject from successfully completing the trial, and participation in any clinical trial within the last three months.

An intravenous line was administered into each subject and a baseline blood sample obtained. The subjects were fasted for 10 hours prior to consuming a 250ml solution comprising one of the four protein ingredients, balanced for leucine content at 2.5 g leucine per 250 ml of solution by adjusting the amount of the protein ingredients added. Consumption of the ingredient was restricted to a 5 minute period.

Following consumption, additional blood samples were obtained at 15, 30, 45, 60, 90, 120 and 180 minute time points.

The plasma was separated and stored at -80°C until analysis. Plasma samples were deproteinised through 100kD filters, and an aliquot of the deproteinised sample transferred into a small tube and vacuum dried. The dried sample was derivatised with Phenyllisothiocyanate (PITC), re-dried under vacuum and reconstituted in a dissolution buffer.

Leucine concentration was measured using reverse phase liquid chromatography with detection at 254nm.

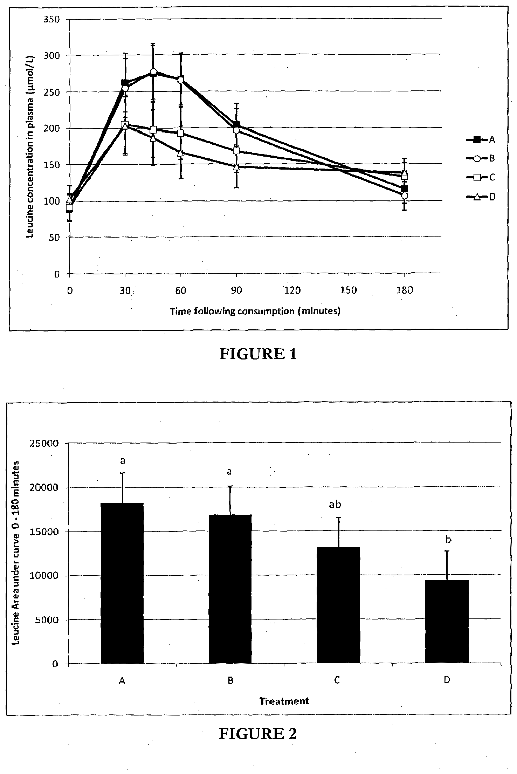

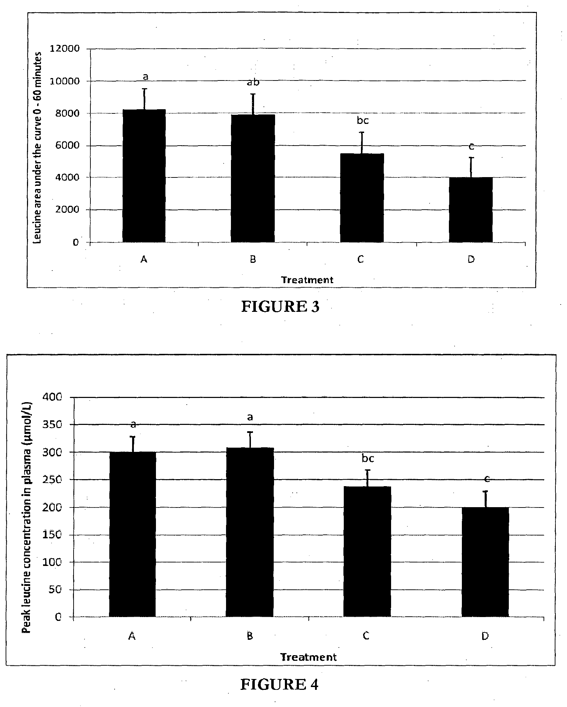

The results are shown in Figures 1 to 4 and show that the greatest plasma concentration of leucine was achieved following consumption of the calcium-depleted milk protein concentrate (A). Levels of leucine were comparable to those achieved following consumption of the standard whey protein concentrate (B). At 30 minutes after consumption, the plasma concentration of standard milk protein concentrate (C) and CaCas (D) had reached a peak, while plasma leucine concentrations still rose for the calcium depleted milk protein concentrate and the whey protein concentrate. The maximum concentration of plasma leucine was achieved 45 minutes after consumption of the calcium depleted milk protein concentrate and the whey protein concentrate. This concentration was significantly different to the concentration of plasma leucine achieved following consumption of CaCas (p<0.001). Additionally, plasma leucine concentration was sustained at a greater level for a longer period following consumption, compared to the other protein ingredients. Once again, the concentration of plasma leucine was significantly greater for the calcium depleted milk protein concentrate and the whey protein concentrate compared to CaCas.

The maximum leucine concentration following consumption of calcium-depleted MPC (A) and WPC (B) was significantly different to the concentration of plasma leucine achieved following consumption of standard MPC (C) (p<0.012) or Calcium Caseinate (D) (p<0.0001). The availability of amino acids in the first 180 minutes, as assessed by area under the curve calculation was significantly greater following the consumption of calcium -depleted MPC and the WPC than the Calcium Caseinate (p<0.0051). The availability of amino acids in the first 60 minutes, as assessed by area under the curve calculation was significantly greater following the consumption of calcium -depleted MPC than either standard MPC (p=0.0123) or Calcium Caseinate (p<0.0001) and significantly different between the WPC and Calcium Caseinate (p<0.0001).

**Example 2: The efficacy of casein compositions for delivering leucine to the bloodstream and for increasing muscle mass**

This example describes a randomised clinical trial for assessing the efficacy of casein compositions for delivering leucine to a subject. The efficacy of the composition for increasing muscle mass in a subject will also be described.

Participants for the trial will be selected according to the inclusion and exclusion criteria detailed in Example 1. All participants will be randomly placed into groups and fed a single dose of a treatment composition.

Treatment compositions may include an $\alpha$-casein, $\beta$-casein or kappa-casein fraction, calcium reduced casein compositions, sodium caseinate, casein in an acidic beverage, casein at a neutral pH with whey or milk protein concentration with normal calcium levels as positive controls.

The primary outcome of the trial will be to assess plasma leucine concentration following consumption of the various protein compositions. Secondary outcomes include assessment of isoleucine and valine plasma concentrations, gastric emptying rate (measured with ultrasonography) and acute gastrointestinal affects following consumption of the protein compositions. Serum amino acid concentrations will be determined by the method of Bloomfield, F.H. et al, (Effects of Intrauterine Growth Restriction and Intraamniotic Insulin-like Growth Factor-I Treatment on Blood and Amniotic Fluid Concentrations and on Fetal Gut Uptake of Amino Acids in Late-Gestation Ovine Fetuses, J. Ped. Gast. Nutr. (2002) 35:287-297), incorporated herein by reference.

Data will show increased plasma leucine concentration and improved gastric emptying in subjects fed the test compositions. The plasma leucine concentrations achieved are comparable to the positive control and better than the negative control compositions.

**Example 3: The efficacy of casein compositions for delivering leucine to the bloodstream**

Each of the protein ingredients were prepared as described above, except sodium caseinate was used rather than calcium caseinate.

Twenty subjects aged 18-49 were selected to participate in the trial. Exclusion criteria for the trial included a regular use of protein supplements, a BMI of greater than $30kg/m^2$, a history of diabetes mellitus - Type I or II, or heart disease, chronic disease of the gastrointestinal tract (for example, inflammatory bowel disease), previous gastrointestinal surgery, pregnancy, allergies to any dietary protein (such as dairy or soy), serious co-morbidity that could, in the opinion of the investigator, prevent the subject from successfully completing the trial, and participation in any clinical trial within the last three months.

An intravenous line was administered into each subject and a baseline blood sample obtained. The subjects were fasted for 10 hours prior to consuming a 250ml solution comprising one of the four protein ingredients, balanced for leucine content at 2.5 g leucine per 250 ml of solution by adjusting the amount of the protein ingredients added. Consumption of the ingredient was restricted to a 5 minute period.

Following consumption, additional blood samples were obtained at 0, 45, and 90 minute time points.

The plasma was separated and stored at -80°C until analysis. Plasma samples were deproteinised through 100kD filters, and an aliquot of the deproteinised sample transferred into a small tube and vacuum dried. The dried sample was derivatised with Phenylisothiocyanate (PITC), re-dried under vacuum and reconstituted in a dissolution buffer.

Amino acid concentrations were measured using reverse phase liquid chromatography with detection at 254nm.

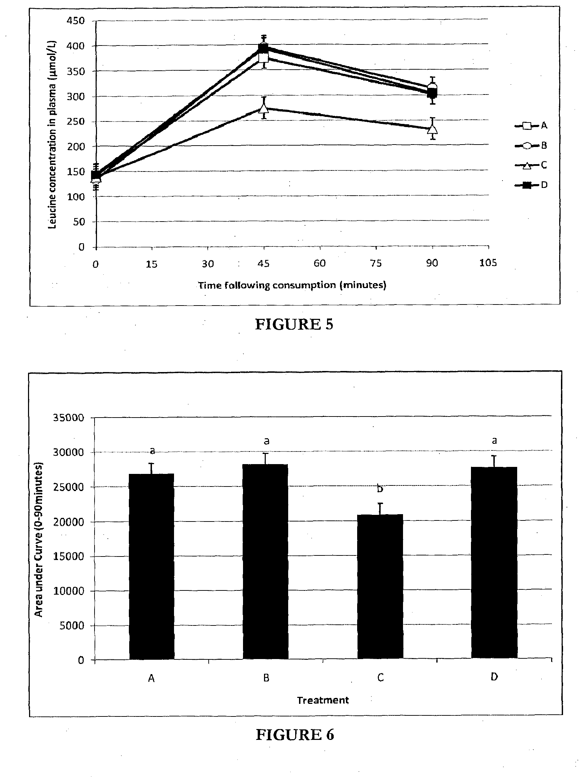

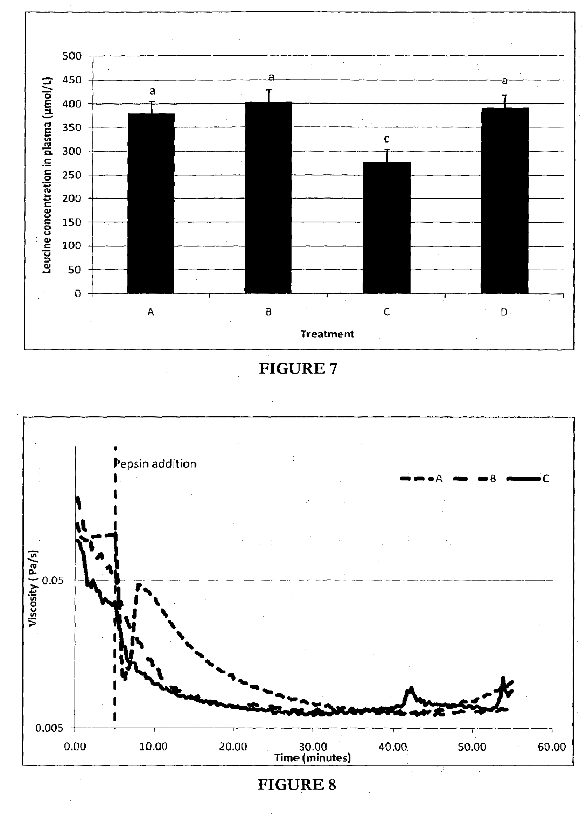

The results are shown in Figures 5 to 7 and show that the greatest peak plasma concentration of leucine was achieved following consumption of the calcium-depleted milk protein concentrate (B) and sodium caseinate (D). Levels of leucine were comparable to those achieved following consumption of the standard whey protein concentrate (A). The maximum concentration was significandy different to the concentration of plasma leucine achieved following consumption of standard MPC (C) (p<0.001) (Figure 7). The availability of amino acids in the first 90 minutes, as assessed by area under the curve calculation was significantly greater following the consumption of calcium -depleted milk protein concentrate, whey protein concentrate and sodium caseinate than standard milk protein concentrate (p<0.05) (Figure 6).

**Example 4: Clotting in a simulated gastric model**

Simulated gastric fluid (SGF) was prepared (US Pharmacopeia Convention, 1995) and warmed to 37°C. Viscosity was measured at 20°C using a rheometer such as an Anton Paar instrument using a stainless steel vane Shear rate = $75s^{-1}$. Protein solutions were prepared by stirring the powder at 50°C for one hour to give a fully hydrated solution. Equal volumes of 37°C protein solution and SGF were mixed and the solution pH was lowered to 1.2 by adding either 6M HCl or 1M HCl. The mixtures were swirled while the acid was added. The mixture was added to the rheometer cup and the stirring started. The viscosity was measured for 5 minutes then pepsin added (E:S ratio of 1:40). The viscosity was then monitored for a further 50 minutes. After 50 minutes, the contents of the sample cup were discarded. As shown in Figure 8, minor differences were observed between sodium caseinate (A), calcium-depleted MPC (B) or standard MPC (C) for viscosity. These differences disappeared within 30 minutes of pepsin addition.

Those persons skilled in the art will understand that the above description is provided by way of illustration only and that the invention is not limited thereto.

**Claims**

1. Use of a casein composition for increasing the blood serum concentration of free leucine in a subject, wherein the casein composition comprises or the casein is in the form of a calcium depleted milk protein concentrate or isolate (MPC or MPI), the casein being 10 to 100% calcium depleted, having a degree of hydrolysis less than 1% and having an unmodified phosphorylation pattern; and wherein the use is not for the purpose of carrying out therapy on the human or animal body.

2. Use of a casein composition for increasing the rate of gastric emptying following ingestion of the composition, increasing the digestibility of a protein composition, or increasing the rate of delivery of amino acids to the blood, wherein the casein composition comprises or the casein is in the form of a calcium depleted milk protein concentrate or isolate (MPC or MPI), the casein being 10 to 100% calcium depleted, having a degree of hydrolysis less than 1% and having an unmodified phosphorylation pattern; and wherein the use is not for the purpose of carrying out therapy on the human or animal body.

3. A use of any one of the preceding claims wherein the casein increases the blood serum concentration of free leucine in a subject within 15, 30, 45 or 60 minutes of administration.

4. A use of any one of the preceding claims wherein the casein is able to increase the concentration of free leucine in blood serum to at least 205 to 400 μmol/L.

5. A use of any one of the preceding claims wherein the casein is able to increase the concentration of free leucine in blood serum to at least 205 μmol/L for at least 5 to 50 minutes.

6. A use of any one of the preceding claims wherein the casein is able to increase the concentration of free leucine in blood serum by at least 105 to 400 μmol/L.

7. A use of any one of the preceding claims wherein the casein is able to increase the concentration of free leucine in blood serum by at least 105 μmol/L for at least 5 to 50 minutes.

8. A use of any one of the preceding claims wherein the casein remains soluble at a pH of 1 to 5, remains soluble in the gastrointestinal tract, remains soluble in gastrointestinal fluid, does not form a coagulum at a pH of 1 to 5, or does not form a coagulum in the gastrointestinal tract.

9. A use of any one of the preceding claims wherein the casein is substantially free of bound calcium.

10. A use of any one of the preceding claims wherein the casein composition comprises calcium depleted casein composition; calcium depleted skim milk; calcium depleted skim milk powder; calcium depleted whole milk; calcium depleted whole milk powder; caseinate, sodium caseinate, potassium caseinate, zinc caseinate, magnesium caseinate; a milk protein concentrate or isolate that has been modified to dissociate casein micelles; non-micellar casein; a casein ingredient, such as an MPC or MPI, where at least a portion of the calcium or phosphate or both the calcium and phosphate has been replaced with sodium, potassium, zinc, magnesium and like, or a combination of any two or more thereof; an acid soluble casein; a non-micellar caseinate; chelated casein micelles; a charge-modified casein; a glycosylated casein; a casein modified to decrease its ability to form a coagulum at acid pH, preferably at a pH below pH 5, pH 4, or pH 3; a casein modified to speed its ability to be hydrolysed in the gastrointestinal tract; a casein ingredient with an altered ratio of casein to whey; a lactic acid casein; a mineral acid casein; one or more casein fractions; an alpha-casein fraction; a beta-casein fraction; a kappa-casein fraction; or any combination of any two or more thereof.

11. A use of any one of the preceding claims wherein the casein is stable at a pH of 1 to 5.

12. A use of any one of the preceding claims wherein the increased blood serum leucine concentration results in any one or more of the following:

   a) maintained or increased muscle protein synthesis,
   b) maintained or increased muscle mass,
   c) prevention or reduction of loss of muscle mass,

d) maintained or increased growth,

e) preventing or decreasing muscle catabolism,

f) increased rate of glycogen resynthesis,

g) modulation of blood sugar levels,

h) increased insulin response to raised blood glucose concentration,

i) increased satiety,

j) reduced satiation,

k) reduced food intake,

l) reduced calorie intake,

m) improved glucose metabolism,

n) increased rate of recovery following exercise, or

o) increased sports performance.

13. A use of any one of the preceding claims wherein the composition comprises at least 0.01 to 99% by weight casein or the composition comprises 0.01 to 99% by weight protein and the protein comprises 0.01 to 99% by weight casein.

14. A use of any one of the preceding claims wherein the casein contains 0 to 3 g calcium/100 g casein.

15. A casein composition for the use in a)preventing or treating cachexia in a subject, b)preventing or treating sarcopenia in a subject, c)increasing the rate of recovery following surgery in a subject, or d)increasing the rate of recovery following injury in a subject, by increasing the blood serum concentration of free leucine in the subject;
wherein the casein is 10 to 100% calcium depleted, has a degree of hydrolysis less than 1% and has an unmodified phosphorylation pattern; and
wherein the casein composition comprises or the casein is in the form of a calcium depleted milk protein concentrate or isolate (MPC or MPI).

**Patentansprüche**

1. Verwendung einer Kaseinzusammensetzung zur Steigerung der Blutserumkonzentration von freiem Leucin in einem Subjekt, wobei die Kaseinzusammensetzung ein calciumdepletiertes Milchproteinkonzentrat oder -isolat (MPC oder MPI) umfasst oder das Kasein in der Form davon ist, wobei das Kasein 10 bis 100 % calciumdepletiert ist, einen Hydrolysegrad von weniger als 1 % hat und ein unmodifiziertes Phosphorylierungsmuster hat; und wobei die Verwendung nicht dem Zweck der Durchführung einer Therapie am menschlichen oder tierischen Körper dient.

2. Verwendung einer Kaseinzusammensetzung zur Steigerung der Rate der Magenentleerung nach Einnahme der Zusammensetzung, Steigerung der Verdaulichkeit einer Proteinzusammensetzung oder Steigerung der Rate der Zufuhr von Aminosäuren ins Blut, wobei die Kaseinzusammensetzung ein calciumdepletiertes Milchproteinkonzentrat oder - isolat (MPC oder MPI) umfasst oder das Kasein in der Form davon ist, wobei das Kasein 10 bis 100 % calciumdepletiert ist, einen Hydrolysegrad von weniger als 1 % hat und ein unmodifiziertes Phosphorylierungsmuster hat; und wobei die Verwendung nicht dem Zweck der Durchführung einer Therapie am menschlichen oder tierischen Körper dient.

3. Verwendung nach einem der vorherigen Ansprüche, wobei das Kasein die Blutserumkonzentration von freiem Leucin in einem Subjekt innerhalb von 15, 30, 45 oder 60 Minuten nach Verabreichung erhöht.

4. Verwendung nach einem der vorherigen Ansprüche, wobei das Kasein die Konzentration von freiem Leucin im Blutserum auf mindestens 205 bis 400 $\mu$mol/L erhöhen kann.

5. Verwendung nach einem der vorherigen Ansprüche, wobei das Kasein die Konzentration von freiem Leucin im Blutserum auf mindestens 205 $\mu$mol/L für mindestens 5 bis 50 Minuten erhöhen kann.

6. Verwendung nach einem der vorherigen Ansprüche, wobei das Kasein die Konzentration von freiem Leucin im Blutserum um mindestens 105 auf 400 $\mu$mol/L erhöhen kann.

7. Verwendung nach einem der vorherigen Ansprüche, wobei das Kasein die Konzentration von freiem Leucin im Blutserum um mindestens 105 $\mu$mol/L für mindestens 5 bis 50 Minuten erhöhen kann.

8. Verwendung nach einem der vorherigen Ansprüche, wobei das Kasein bei einem pH-Wert von 1 bis 5 löslich bleibt, im Verdauungstrakt löslich bleibt, in Gastrointestinalflüssigkeit löslich bleibt, kein Blutgerinnsel bei einem pH-Wert von 1 bis 5 bildet oder kein Blutgerinnsel im Verdauungstrakt bildet.

9. Verwendung nach einem der vorherigen Ansprüche, wobei das Kasein im Wesentlichen frei von gebundenem Calcium ist.

10. Verwendung nach einem der vorherigen Ansprüche, wobei die Kaseinzusammensetzung umfasst: calciumdepletierte Kaseinzusammensetzung; calciumdepletierte Magermilch; calciumdepletiertes Magermilchpulver; calciumdepletierte Vollmilch; calciumdepletiertes Vollmilchpulver; Kaseinat, Natriumkaseinat, Kaliumkaseinat, Zinkkaseinat, Magnesiumkaseinat; ein Milchproteinkonzentrat oder -isolat, das dazu modifiziert wurde, Kasein-Mizellen zu trennen; nicht-mizellares Kasein; einen Kasein-Bestandteil, wie MPC oder MPI, bei dem mindestens ein Teil des Calciums oder des Phosphats oder des Calciums und des Phosphats durch Natrium, Kalium, Zink, Magnesium und dergleichen oder einer Kombination aus zwei oder mehreren davon ersetzt wurde; ein säurelösliches Kasein; ein nicht-mizellares Kaseinat; chelatierte Kasein-Mizellen; ein ladungsmodifiziertes Kasein; ein glykosyliertes Kasein; ein Kasein, das dazu modifiziert ist, seine Fähigkeit zum Bilden eines Blutgerinnsels bei einem sauren pH-Wert, vorzugsweise bei einem pH-Wert unter 5, 4 oder 3, zu verringern; ein Kasein, das dazu modifiziert ist, seine Fähigkeit, im Verdauungstrakt hydrolysiert zu werden, zu beschleunigen; einen Kasein-Bestandteil mit einem veränderten Verhältnis von Kasein zu Molke; ein Milchsäure-Kasein; ein Mineralsäure-Kasein; einen oder mehrere Kasein-Bruchteile; einen Alpha-Kasein-Bruchteil; einen Beta-Kasein-Bruchteil; einen Kappa-Kasein-Bruchteil; oder eine Kombination aus zwei oder mehreren davon.

11. Verwendung nach einem der vorherigen Ansprüche, wobei das Kasein bei einem pH-Wert von 1 bis 5 stabil ist.

12. Verwendung nach einem der vorherigen Ansprüche, wobei die gesteigerte Blutserum-Leucinkonzentration in einem oder mehreren des Folgenden resultiert:

    (a) beibehaltene oder gesteigerte Muskel-Protein-Synthese,
    (b) beibehaltene oder gesteigerte Muskelmasse,
    (c) Prävention oder Reduktion des Verlustes von Muskelmasse,
    (d) beibehaltenes oder gesteigertes Wachstum,
    (e) Verhindern oder Vermindern des Muskelabbaus,
    (f) gesteigerte Rate der Glykogenresynthese,
    (g) Modulation des Blutzuckerspiegels,
    (h) gesteigerte Insulinreaktion auf erhöhte Blutzuckerkonzentration,
    (i) gesteigerte Sättigung,
    (j) vermindertes Sättigungsgefühl,
    (k) verminderte Nahrungsaufnahme,
    (l) verminderte Kalorienaufnahme,
    (m) verbesserter Glukosestoffwechsel,
    (n) gesteigerte Erholungsrate nach Bewegung oder
    (o) gesteigerte sportliche Leistungsfähigkeit.

13. Verwendung nach einem der vorherigen Ansprüche, wobei die Zusammensetzung mindestens 0,01 bis 99 % Kasein nach Gewicht umfasst oder die Zusammensetzung 0,01 bis 99 % Protein nach Gewicht umfasst und das Protein 0,01 bis 99 % Kasein nach Gewicht umfasst.

14. Verwendung nach einem der vorherigen Ansprüche, wobei das Kasein 0 bis 3 g Calcium/100 g Kasein enthält.

15. Kaseinzusammensetzung zur Verwendung bei der a) Prävention oder Behandlung von Kachexie in einem Subjekt, b) Prävention oder Behandlung von Sarkopenie in einem Subjekt, c) Steigerung der Erholungsrate nach einer Operation in einem Subjekt oder d) Steigerung der Erholungsrate nach einer Verletzung in einem Subjekt durch Steigerung der Blutserumkonzentration von freiem Leucin in dem Subjekt;
wobei das Kasein 10 bis 100 % calciumdepletiert ist, einen Hydrolysegrad von weniger als 1 % hat und ein unmodifiziertes Phosphorylierungsmuster hat; und
wobei die Kaseinzusammensetzung ein calciumdepletiertes Milchproteinkonzentrat oder -isolat (MPC oder MPI) umfasst oder das Kasein in der Form davon ist.

**Revendications**

1. Utilisation d'une composition caséinique pour augmenter la concentration en leucine libre dans le sérum sanguin d'un sujet, dans laquelle la composition caséinique comprend un concentré ou isolé de protéine laitière (MPC ou MPI) appauvri en calcium, ou la caséine est sous la forme dudit concentré ou isolé, la caséine étant de 10 à 100 % appauvrie en calcium, ayant un degré d'hydrolyse inférieur à 1 % et ayant un motif de phosphorylation non modifié ; et dans laquelle l'utilisation n'est pas dans le but d'effectuer une thérapie sur le corps humain ou animal.

2. Utilisation d'une composition caséinique pour augmenter le taux de vidange gastrique suivant l'ingestion de la composition, augmenter la digestibilité d'une composition de protéine, ou augmenter le taux d'apport en acides aminés au sang, dans laquelle la composition caséinique comprend un concentré ou isolé de protéine laitière (MPC ou MPI) appauvri en calcium, ou la caséine est sous la forme dudit concentré ou isolé, la caséine étant de 10 à 100% appauvrie en calcium, ayant un degré d'hydrolyse inférieur à 1 % et ayant un motif de phosphorylation non modifié ; et dans laquelle l'utilisation n'est pas dans le but de réaliser une thérapie sur le corps humain ou animal.

3. Utilisation selon l'une quelconque des revendications précédentes, dans laquelle la caséine augmente la concentration en leucine libre dans le sérum sanguin d'un sujet au sein de 15, de 30, de 45 ou de 60 minutes de l'administration.

**4.** Utilisation selon l'une quelconque des revendications précédentes, dans laquelle la caséine est capable d'augmenter la concentration en leucine libre dans le sérum sanguin à au moins 205 à 400 µmol/L.

**5.** Utilisation selon l'une quelconque des revendications précédentes, dans laquelle la caséine est capable d'augmenter la concentration en leucine libre dans le sérum sanguin à au moins 205 µmol/L pendant au moins 5 à 50 minutes.

**6.** Utilisation selon l'une quelconque des revendications précédentes, dans laquelle la caséine est capable d'augmenter la concentration en leucine libre dans le sérum sanguin d'au moins 105 à 400 µmol/L.

**7.** Utilisation selon l'une quelconque des revendications précédentes, dans laquelle la caséine est capable d'augmenter la concentration en leucine libre dans le sérum sanguin d'au moins 105 µmol/L pendant au moins 5 à 50 minutes.

**8.** Utilisation selon l'une quelconque des revendications précédentes, dans laquelle la caséine reste soluble à un pH d'1 à 5, reste soluble dans le tube gastrointestinal, reste soluble dans un fluide gastrointestinal, ne forme pas de coagulum à un pH d'1 à 5, ou ne forme pas de coagulum dans le tube gastrointestinal.

**9.** Utilisation selon l'une quelconque des revendications précédentes, dans laquelle la caséine est sensiblement dépourvue de calcium lié.

**10.** Utilisation selon l'une quelconque des revendications précédentes, dans laquelle la composition caséinique comprend une composition caséinique appauvrie en calcium ; du lait écrémé appauvri en calcium ; de la poudre de lait écrémé appauvri en calcium ; du lait entier appauvri en calcium ; de la poudre de lait entier appauvri en calcium ; du caséinate, du caséinate de sodium, du caséinate de potassium, du caséinate de zinc, du caséinate de magnésium ; un concentré ou isolé de protéine laitière qui a été modifié pour dissocier des micelles caséiniques ; de la caséine non micellaire ; un ingrédient caséinique, tel qu'un MPC ou MPI, où au moins une portion du calcium ou du phosphate, ou à la fois du calcium et du phosphate, a été remplacée avec du sodium, du potassium, du zinc, du magnésium et analogues, ou une association de deux ou plus quelconques de ceux-ci ; une caséine soluble en acide ; un caséinate non micellaire ; des micelles caséiniques chélatées ; une caséine modifiée en charge ; une caséine glycosylée ; une caséine modifié pour réduire sa capacité de former un coagulum à un pH acide, de préférence à un pH inférieur à pH 5, pH 4, ou pH 3 ; une caséine modifiée pour accélérer sa capacité d'être hydrolysée dans le tube gastrointestinal ; un ingrédient caséinique avec un rapport caséine-lactosérum modifié ; une caséine d'acide lactique ; une caséine d'acide minéral ; une ou plusieurs fractions caséiniques ; une fraction caséinique alpha ; une fraction caséinique bêta ; une fraction caséinique kappa ; ou une quelconque association de deux ou plus quelconque de ceux-ci.

**11.** Utilisation selon l'une quelconque des revendications précédentes, dans laquelle la caséine est stable à un pH de 1 à 5.

**12.** Utilisation selon l'une quelconque des revendications précédentes, dans laquelle la concentration augmentée en leucine dans le sérum sanguin a pour résultat un ou plusieurs quelconques de ce qui suit :

    a) une synthèse protéinique musculaire maintenue ou accrue,
    b) une masse musculaire maintenue ou accrue,
    c) la prévention ou réduction de perte de masse musculaire,
    d) une croissance maintenue ou accrue,
    e) la prévention ou réduction de catabolisme musculaire,
    f) un taux accru de resynthèse glycogénique,
    g) la modulation de taux de glycémie,
    h) une réponse insulinique accrue à une concentration glycémique accrue,
    i) une satiété accrue,
    j) une satiation réduite,
    k) un apport alimentaire réduit,
    l) un apport calorique réduit,
    m) un métabolisme glycémique amélioré,
    n) un taux accru de rétablissement après exercice, ou
    o) une performance sportive accrue.

**13.** Utilisation selon l'une quelconque des revendications précédentes dans laquelle la composition comprend au moins de 0,01 à 99 % en poids de caséine ou la composition comprend de 0,01 à 99 % en poids de protéine et la protéine comprend de 0,01 à 99 % en poids de caséine.

**14.** Utilisation selon l'une quelconque des revendications précédentes dans laquelle la caséine contient de 0 à 3 g de calcium/100 g de caséine.

**15.** Composition caséinique pour l'utilisation dans a) la prévention ou le traitement de cachexie chez un sujet, b) la prévention ou le traitement de sarcopénie chez un sujet, c) augmentant le taux de récupération suivant une chirurgie chez un sujet, ou d) augmentant le taux de récupération suivant une lésion chez un sujet, en augmentant la concentration de sérum sanguin en leucine libre chez le sujet ;

dans laquelle la caséine est de 10 à 100% appauvrie en calcium, a un degré d'hydrolyse inférieur à 1 % et a un motif de phosphorylation non modifié ; et dans laquelle la composition caséinique comprend ou la caséine est sous la forme de concentré ou d'isolé de protéine laitière (MPC ou MPI) appauvri en calcium.

FIGURE 1

FIGURE 2

EP 2 822 568 B1

FIGURE 3

FIGURE 4

**FIGURE 5**

**FIGURE 6**

**FIGURE 7**

**FIGURE 8**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2001041578 A
- WO 2004057971 A
- WO 2004091309 A

- WO 2001041579 A
- WO 2010107325 A
- NZ 2010000042 W

**Non-patent literature cited in the description**

- **CRUZ-JENTOFT AJ ; BAEYEN JP ; BAUER JM et al.** Sarcopenia: European consensus on definition and diagnosis. *Age and ageing,* 2010, vol. 39, 412-423
- **WOLF RR et al.** Optimal protein intake in the elderly. *Clin Nutr.,* 2008, vol. 5, 675-684
- **GARLICK PJ.** The role of leucine in the regulation of protein metabolism. *J Nutr,* 2005, vol. 135, 1553S-1556S
- Advanced Dairy Chemistry. Proteins. Kluwer Academic/Plenum Publishers, 2003, vol. 1
- **BOIRIE, Y. et al.** Slow and fast dietary proteins differently modulate postprandial protein accretion. *Proc. Natl. Acad. Sci.,* 1997, vol. 94 (26), 14930-14935
- **JOSE A. L. CALBET et al.** Gastric emptying, gastric secretion and enterogastrone response after administration of milk proteins or their peptide hydrolysates in humans. *EUROPEAN JOURNAL OF NUTRITION,* 01 June 2004, vol. 43 (3), 127-139
- **M FOX et al.** Dietary protein precipitation properties have effects on gastric emptying in healthy volunteers. *CLINICAL NUTRITION.,* 01 August 2004, vol. 23 (4), 641-646
- **K. VAN NORREN.** Dose-dependent effects of leucine supplementation on preservation of muscle mass in cancer cachectic mice. *Oncology Reports,* 18 April 2011
- **LAYNE E NORTON et al.** Leucine regulates translation initiation of protein synthesis in skeletal muscle after exercise. *The Journal of Nutrition,* 01 February 2006, 533S-537S
- **ISABELLE RIEU et al.** Leucine supplementation improves muscle protein synthesis in elderly men independently of hyperaminoacidaemia: Leucine and protein metabolism in the elderly. *The Journal of Physiology,* 08 August 2006, vol. 575 (1), 305-315

- **FREIREICH EJ ; GEHAN EA ; RALL DP ; SCHMIDT LH ; SKIPPER HE.** Quantitative comparison of toxicity to anticancer agents in mouse, rat, hamster, dog, monkey and man. *Cancer Chemother Rep,* 1966, vol. 50, 219-244
- Scientific Tables, Geigy Pharmaceuticals. Ardley, 1970, 537
- **PHILIPPE, M. et al.** The effects of different cations on the physicochemical characteristics of casein micelles. *Food Chemistry,* 2005, vol. 90, 673-683
- Tetra Pak Processing Systems AB. Dairy Processing Handbook, 2003 (Dairy Processing Handbook. 2003
- **FOX ; MCSWEENEY.** *Dairy Processing Handbook,* 2003
- United States National Academy of Sciences. Institute of Medicine, Food and Nutrition Board, 2010
- Remington's Pharmaceutical Sciences. Mack Publishing Co, 1980
- **L. A. SZARKA ; M. CAMILLERI.** Methods for measurement of gastric motility. *Am J Physiol Gastrointest Liver Physiol,* 2009, vol. 296, G461-G475
- **A. J. DARRAGH ; S. M. HODGKINSON.** Quantifying die Digestibility of Dietary Protein. *J Nutr.,* July 2000, vol. 130 (7), 1850S-6S
- **BLOOMFIELD, F.H. et al.** Effects of Intrauterine Growth Restriction and Intraamniotic Insulin-like Growth Factor-I Treatment on Blood and Amniotic Fluid Concentrations and on Fetal Gut Uptake of Amino Acids in Late-Gestation Ovine Fetuses. *J. Ped. Gast. Nutr.,* 2002, vol. 35, 287-297